# EUROPEAN PATENT SPECIFICATION

(11) **EP 3 543 344 B1**
(45) Date of publication and mention of the grant of the patent: **28.06.2023**
(21) Application number: 18382195.8
(22) Date of filing: 22.03.2018
(51) Int. Cl.: C12P 7/16, C12P 1/04

(54) **PROCEDURE FOR OBTAINING 2,3-BUTANEDIOL BY FERMENTATION OF HYDROLYZED SUBSTRATES OF ORGANIC WASTE (OW) WITH MICROORGANISMS**
VERFAHREN ZUR GEWINNUNG VON 2,3-BUTANDIOL DURCH FERMENTATION VON HYDROLYSIERTEN SUBSTRATEN AUS ORGANISCHEN ABFÄLLEN (OA) MIT MIKROORGANISMEN
PROCEDE D'OBTENTION DE 2,3-BUTANEDIOL PAR FERMENTATION DE SUBSTRATS HYDROLYSÉS DE DÉCHETS ORGANIQUES (DO) AVEC DES MICRO-ORGANISMES

(43) Date of publication of application: 25.09.2019
(73) Proprietor: Biopolis, S.L., 46980 Paterna (Valencia) (ES); S.A. Agricultores de la Vega de Valencia, 46003 Valencia (ES)
(72) Inventor: BUSTAMANTE JARAMILLO, Daniel Arturo, 28015 Madrid (ES); SANCHIS AMAT, María, 46008 Valencia (ES); ROJAS MARTINEZ, Antonia María, 46015 Valencia (ES); TORTAJADA SERRA, Marta, 46015 Valencia (ES); RAMÓN VIDAL, Daniel, 46183 La Eliana (Valencia) (ES); FERRER PALOMARES, Lilian, 46025 Valencia (ES); SALA VILLAPLANA, Francisco, 46007 Valencia (ES); PASCÓ SERRA, José Ignacio, 46007 Valencia (ES)
(74) Representative: Pons

(56) References cited:
- WO-A1-2018/019656
- JP-A- 2012 105 635
- US-A1- 2013 316 418
- US-A1- 2015 191 752

## Description

This invention refers to a procedure for obtaining 2,3-Butanediol by fermenting the hydrolyzed substrates of solid or liquid waste, of any origin, with high or medium contents of organic matter: organic waste (hereinafter OW) using microorganisms, particularly by using a strain of *Raoultella planticola.*

### STATE OF THE ART

The compound 2,3-Butanediol (2,3-BD) is a compound of interest as a chemical reagent for use in different types of industries thanks to its unusual stereochemistry. It has 3 isomers: dextro-, levo- and meso-; and all three are produced by microbial fermentation.

Levo-isomer freezes at -60ºC, which makes it commercially interesting as antifreeze.

Both dextro- and levo-isomers are excellent chiral components for asymmetric synthesis and are used in the pharmaceutical, agrochemical, fine chemical and food industries, and as liquid crystals.

2,3-BD is easily dehydrated to methyl-ethyl-ketone (MEK), an excellent organic solvent for resins and lacquers; and to butadiene, for the production of polyesters and polyurethanes.

2,3-BD can also be easily dehydrogenated in acetoin and diacetyl, which are flavoring agents used in the dairy, margarine and cosmetic industries.

2,3-BD has potential applications in the production of inks, perfumes, softening and moisturizing agents, explosives and plasticizers (Zeng A.-P. and Sabra W. (2011) Curr. Op. Biotechnol 22: 749-757).

2,3-BD fermentation has the following advantages:
- It is less toxic than ethanol and other alcohols, thus the culture can reach higher concentrations.
- It is possible to use the main sugars (hexoses and pentoses) obtained in processes of enzymatic hydrolysis of ligno-cellulosic substrates as a carbon source.
- The chemical way of obtaining 2,3-BD is more expensive.

The state of the art describes processes of enzymatically treating biomass to obtain hydrolysates rich in fermentable sugars for their use in processes to obtain chemicals and their industrial scale-up (see WO 2014013330, US7781191 and US7807419).

In addition, processes to obtain 2,3-Butanediol (2, 3-BD) based on the use of *Bacillus licheniformis* as biocatalyst and raw lignocellulose material as substrate (see CN103497917) have been reported. The strain used in CN103497917 can produce 2,3-Butanediol at 43-55ºC with a conversion of greater than 90% of the theoretical conversion rate. The concentration of 2,3-Butanediol can reach 92 g/L, with a production efficiency of 1.1 g/L/h.

Therefore, it would be desirable to have a procedure to obtain 2,3-BD using OW as substrate based on the use of microorganisms that would enable a greater amount of 2,3-BD to be obtained.

### DESCRIPTION OF THE INVENTION

One aspect of this invention refers to the procedure for obtaining 2,3-Butanediol from a substrate of hydrolyzed OW (organic waste) that comprises fermentation of the substrate with a strain of *Raoultella planticola,*
wherein:
OW is MSW (municipal solid waste), BMSW (biodegradable fraction of municipal solid wastes, stabilized or unstabilized), sewage sludge from WWPP (waste water purification plants), agricultural and livestock waste (such as slurry), waste from the agro-alimentary industry, and mixtures thereof, characterized in that OW is generated in urban areas,
the strain of *Raoultella planticola* is CECT843, CECT8158 or CECT8159, the hydrolysate of OW is used as a substrate at a concentration of between 50% and 100%, and
the OW substrate is formulated with the culture media M1, M2 or M3, wherein:
   M1 consisting of 4.4 g/L K₂HPO₄, 1.3 g/L KH₂PO₄, 2 g/L (NH₄)₂SO₄, 0.2 g/L MgSO₄, 1.0 g/L yeast extract, 1 mUL trace elements solution [70 mg/L ZnCl₂, 0.1 g/L MnCl₂·4H₂O, 60 mg/L H₃BO₃, 0.2 g CoCl₂H₂0, 20 mg CuCl₂·2H₂O, 25 mg NiCl₂·6H₂O, 35 mg Na₂MoO₄·2H₂O, 0.9 mL/L HCl];
   M2 consisting of 5.0 g/L yeast extract, 5.0 g/L triptone, 7.0 g/L KH₂PO₄, 7.0 g/L K₂HPO₄, 1.0 g/L (NH₄)₂SO₄, 0.25 g/L MgSO₄·7H₂O, 0.12 g/L NaMoO₄·7H₂O, 0.021 g/L CaCl₂·2H₂O, 0.029 g/L CoCl₂·6H₂O, 0.039 g/L Fe(NH₂)₂SO₄·6H₂O, 2 mg/L nicotininic acid, 0.172 mg/L Na₂SeO₃, 0.02 mg/L NiCl₂, 10 mL/L trace elements solution [0.5 g/L Na₂EDTA, 0.5 g/L MnCl₂·4H₂O, 0.1 g/L H₃BO₃, 1.0 mg/L CuCl₂·2H₂O]; and
   M3 consisting of 0.75 g/L KCI, 1.38 g/L NaH₂PO₄·2H₂O, 5.35 g/L (NH₄)₂SO₄, 0.28 g/L Na₂SO₄, 0.26 g/L MgSO₄·7H₂O, 0.42 g/L citric acid, 2 g/L yeast extract, 0.3 mL/L trace elements solution [34.2 g/L ZnCl₂, 2.7 g/L FeCl₃·6H₂O, 10 g/L MnCl₂·4H₂O, 0.85 g/L CuCl₂·2H₂O, 0.3 g/L H₃BO₃, 23.8 g/L CoCl₂·6H₂O].

Another realization of the invention refers to the procedure described above, where:
- the strain of *Raoultella planticola* is CECT843; and
- hydrolyzed OW is used as a substrate at a concentration of between 50% and 100%, and preferably between 75% and 80%.

Another realization of the invention refers to the procedure described above, where:
- the strain of *Raoultella planticola* is strain CECT8158 or strain CECT8159; and
- hydrolyzed OW is used as a substrate at a concentration of between 50% and 100%, and preferably between 75% and 80%.

Throughout this invention, the culture media M1, M2 and M3 refer to:
**Medium 1** [M1, according to Cheng K.-K., Liu Q., Zhag J.-A., Ping J.-M., Wang G.-H. (2010) Process Biochem. 45: 613-616]: 4.4 g/L K₂HPO₄, 1.3 g/L KH₂PO₄, 2 g/L (NH₄)₂SO₄, 0.2 g/L MgSO₄, 1.0 g/L yeast extract, 1 mL/L trace elements solution [70 mg/L ZnCl₂, 0.1 g/L MnCl₂·4H₂O, 60 mg/L H₃BO₃, 0.2 g CoCl₂H₂O, 20 mg CuCl₂·2H₂O, 25 mg NiCl₂·6H₂O, 35 mg Na₂MoO₄·2H₂O, 0.9 mL/L HCl.
**Medium 2** [M2] 5.0 g/L yeast extract, 5.0 g/L triptone, 7.0 g/L KH₂PO₄, 7.0 g/L K₂HPO₄, 1.0 g/L (NH₄)₂SO₄, 0.25 g/L MgSO₄·7H₂O, 0.12 g/L NaMoO₄·7H₂O, 0.021 g/L CaCl₂·2H₂O, 0.029 g/L CoCl₂·6H₂O, 0.039 g/L Fe(NH₂)₂SO₄·6H₂O, 2 mg/L nicotininic acid, 0.172 mg/L Na₂SeO₃, 0.02 mg/L NiCl₂, 10 mL/L trace elements solution [0.5 g/L Na₂EDTA, 0.5 g/L MnCl₂·4H₂O, 0.1 g/L H₃BO₃, 1.0 mg/L CuCl₂·2H₂O.
**Medium 3** [M3, according to Petrov & Petrova (2009) Appl. Microb. Biotechnol. 84: 659-665]: 0.75 g/L KCI, 1.38 g/L NaH₂PO₄·2H₂O, 5.35 g/L (NH₄)₂SO₄, 0.28 g/L Na₂SO₄, 0.26 g/L MgSO₄·7H₂O, 0.42 g/L citric acid, 2 g/L yeast extract, 0.3 mL/L trace elements solution [34.2 g/L ZnCl₂, 2.7 g/L FeCl₃·6H₂O, 10 g/L MnCl₂·4H₂O, 0.85 g/L CuCl₂·2H₂O, 0.3 g/L H₃BO₃, 23.8 g/L CoCl₂·6H₂O]

Another realization of the invention refers to the procedure described above, where:
- the strain of *Raoultella planticola* is CECT843;
- OW hydrolysate is used as a substrate at a concentration of between 50% and 100%, and preferably between 75% and 80%; and
- the OW substrate is formulated with the culture media M1, M2 or M3.

Another realization of the invention refers to the procedure described above, where:
- the strain of *Raoultella planticola* is strain CECT8158 or strain CECT8159;
- OW hydrolysate is used as a substrate at a concentration of between 50% and 100%, and preferably between 75% and 80%; and
- the OW substrate is formulated with the culture media M1, M2 or M3.

In another realization, the invention refers to the procedure described above, where the temperature at which it is performed is between 25°C and 35°C, preferably between 28°C and 32ºC, and more preferably at 30ºC.

Another realization of the invention refers to the procedure described above, where:
- the strain of *Raoultella planticola* is CECT843;
- hydrolyzed OW is used as a substrate at a concentration of between 50% and 100%, and preferably between 75% and 80%.
- The OW substrate is formulated with the culture media M1, M2 or M3; and
- the temperature at which it is performed is between 25°C and 35°C, preferably between 28°C and 32 C, and more preferably at 30ºC.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3; and
- the temperature at which it is performed is between 25°C and 35°C, preferably between 28°C and 32ºC, and more preferably at 30ºC.

In another realization the invention refers to the procedure described above, characterized by being carried out under shaking at between 100 rpm and 400 rpm, and preferably between 200 rpm and 350 rpm.

Another realization of the invention refers to the procedure described above, where:
- the strain of *Raoultella planticola* is CECT843;
- hydrolyzed OW is used as a substrate at a concentration of between 50% and 100%, and preferably between 75% and 80%;
- the OW substrate is formulated with the culture media M1, M2 or M3; and
- performed under shaking at between 100 rpm and 400 rpm, and preferably between 200 rpm and 350 rpm.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3; and
- performed under shaking at between 100 rpm and 400 rpm, and preferably between 200 rpm and 350 rpm.

In another realization the invention refers to the procedure described above, where:
- the strain of *Raoultella planticola* is CECT843;
- hydrolyzed OW is used as substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32ºC, and more preferably at 30ºC; and
- performed under shaking at between 100 rpm and 400 rpm, and preferably between 200 rpm and 350 rpm.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32ºC, and more preferably at 30ºC; and
- performed under shaking at between 100 rpm and 400 rpm, and preferably between 200 rpm and 350 rpm.

In another realization the invention refers to the procedure described above characterized by being carried out at a pH below 7, and preferably at pH 6.5.

Another realization of the invention refers to the procedure described above, where:
- the strain of *Raoultella planticola* is CECT843;
- hydrolyzed OW is used as a substrate at a concentration of between 50% and 100%, and preferably between 75% and 80%;
- the OW substrate is formulated with the culture media M1, M2 or M3; and
- performed at a pH below 7, and preferably at pH 6.5.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3; and
- performed at a pH below 7, and preferably at pH 6.5.

Another realization of the invention refers to the procedure described above, where:
- the strain of *Raoultella planticola* is CECT843;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32ºC, and more preferably at 30ºC;
- performed under shaking at between 100 rpm and 400 rpm, and preferably between 200 rpm and 350 rpm;
- performed at a pH below 7, and preferably at pH 6.5.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32ºC, and more preferably at 30ºC;
- performed under shaking at between 100 rpm and 400 rpm, and preferably between 200 rpm and 350 rpm;
- performed at a pH below 7, and preferably at pH 6.5.

In another realization the invention refers to the procedure described above characterized by being carried out with an aeration of between 0.7 vvm and 2.0 vvm, preferably at 1.5 vvm.

Another realization of the invention refers to the procedure described above, where:
- the strain of *Raoultella planticola* is CECT843;
- hydrolyzed OW is used as a substrate at a concentration of between 50% and 100%, and preferably between 75% and 80%;
- the OW substrate is formulated with the culture media M1, M2 or M3; and
- performed with an aeration of between 0.7 vvm and 2.0 vvm, preferably at 1.5 vvm.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3; and
- performed with an aeration of between 0.7 vvm and 2.0 vvm, preferably at 1.5 vvm.

Another realization of the invention refers to the procedure described above, where:
- the strain of *Raoultella planticola* is CECT843;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32º C, and more preferably at 30ºC;
- performed under shaking at between 100 rpm and 400 rpm, and preferably between 200 rpm and 350 rpm;
- performed at a pH below 7, and preferably at pH 6.5; and
- performed with an aeration of between 0.7 vvm and 2.0 vvm, preferably at 1.5 vvm.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32º C, and more preferably at 30ºC;
- performed under shaking at between 100 rpm and 400 rpm, and preferably between 200 rpm and 350 rpm;
- performed at a pH below 7, and preferably at pH 6.5; and
- performed with an aeration of between 0.7 vvm and 2.0 vvm, preferably at 1.5 vvm.

In another realization the invention refers to the procedure described above, which includes a fed-batch culture feeding step.

In another realization the invention refers to the procedure described above where fed-batch culture feeding is carried out by means of substrate pulses at different time intervals, and preferably at 16 h, 20 h, 24 h and 40 h time intervals.

Another realization of the invention refers to the procedure described above, where:
- the strain of *Raoultella planticola* is CECT843;
- hydrolyzed OW is used as a substrate at a concentration of between 50% and 100%, and preferably between 75% and 80%; and
- comprising a fed-batch culture feeding step, preferably where fed-batch substrate feeding is carried out by means of substrate pulses at different time intervals, and preferably at 16 h, 20 h, 24 h and 40 h time intervals.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%; and
- comprising a fed-batch culture feeding step, preferably where fed-batch substrate feeding is carried out by means of substrate pulses at different time intervals, and preferably at 16 h, 20 h, 24 h and 40 h time intervals.

Another realization of the invention refers to the procedure described above, where:
- the strain of *Raoultella planticola* is CECT843;
- hydrolyzed OW is used as a substrate at a concentration of between 50% and 100%, and preferably between 75% and 80%.
- the OW substrate is formulated with the culture media M1, M2 or M3; and
- comprising a fed-batch culture feeding step, preferably where fed-batch substrate feeding is carried out by means of substrate pulses at different time intervals, and preferably at 16 h, 20 h, 24 h and 40 h time intervals.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%.
- the OW substrate is formulated with the culture media M1, M2 or M3; and
- comprising a fed-batch culture feeding step, preferably where fed-batch substrate feeding is carried out by means of substrate pulses at different time intervals, and preferably at 16 h, 20 h, 24 h and 40 h time intervals.

Another realization of the invention refers to the procedure described above, where:
- the strain of *Raoultella planticola* is CECT843;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32º C, and more preferably at 30ºC;
- performed under shaking at between 100 rpm and 400 rpm, and preferably between 200 rpm and 350 rpm;
- performed at a pH below 7, and preferably at pH 6.5;
- performed with aeration of between 0.7 vvm and 2.0 vvm, preferably at 1.5 vvm; and
- comprising a fed-batch culture feeding step, preferably where fed-batch substrate feeding is carried out by means of substrate pulses at different time intervals, and preferably at 16 h, 20 h, 24 h and 40 h time intervals.

Another realization of the invention refers to the procedure described above, where:
- the strain of *Raoultella planticola* is strain CECT8158 or strain CECT8159;
- hydrolyzed OW is used as a substrate at a concentration of between 50% and 100%, and preferably between 75% and 80%;
- the OW substrate is formulated with the culture media M1, M2 or M3;
- the temperature at which it is performed is between 25°C and 35°C, preferably between 28°C and 32ºC, and more preferably at 30ºC;
- performed under shaking at between 100 rpm and 400 rpm, and preferably between 200 rpm and 350 rpm;
- performed at a pH below 7, and preferably at pH 6.5;
- performed with aeration of between 0.7 vvm and 2.0 vvm, preferably at 1.5 vvm; and
- comprising a fed-batch culture feeding step, preferably where fed-batch substrate feeding is carried out by means of substrate pulses at different time intervals, and preferably at 16 h, 20 h, 24 h and 40 h time intervals.

In the present invention the term "OW" or "organic waste" means organic waste that includes all waste having an organic load derived from human presence or activity, and can be understood therefore as: MSW (municipal solid waste), BMSW (biodegradable fraction of municipal solid wastes, stabilized or unstabilized), sewage sludge from WWPP (waste water purification plants), agricultural and livestock waste (such as slurry), waste from the agro-alimentary industry, as well as the possible mixtures of all the above, and that is generated in urban areas or in their municipalities.

The term "MSW" or "municipal solid waste" refers to a solid that is generated in urban areas or in their municipalities (private homes, shops, offices and services). MSW can contain organic matter, paper and cardboard, plastics and glass.

In the present invention, the term "fed-batch" refers to a continuous or pulsed substrate feeding system.

Throughout the description and claims the word "comprises" and its variants are not intended to exclude other technical characteristics, additives, components or steps. For the experts in the matter, other objects, advantages and characteristics of the invention will be derived partly from the description and partly from the practice of the invention. The following examples and figures are provided as way of example, and are not intended to be restrictive of the present invention.

### BRIEF DESCRIPTION OF THE FIGURES

**Fig. 1****.** The figure shows the fermentation of *Raoultella planticola* CECT843 in *fed-batch* mode with hydrolyzed substrate in a 1.5L bioreactor. The arrows indicate the points at which new substrate was added without concentration.
**Fig. 2****.** The figure shows the fermentation of *Raoultella planticola* CECT843 in *fed-batch* mode in a 1.5L bioreactor with concentrated hydrolyzed substrate.
**Fig. 3****.** The figure shows the optimization of *Raoultella planticola* CECT843 fermentation with concentrated hydrolyzed substrate in *fed-batch* mode in a 1.5L bioreactor.
**Fig. 4****.** The figure shows the fermentation of *Raoultella planticola* CECT843 in *fed-batch* mode with concentrated hydrolyzed substrate in a 20L bioreactor.
**Fig. 5****.** The figure shows the fermentation of *Raoultella planticola* CECT 843 in *fed-batch* mode with concentrated hydrolyzed substrate (compost) in a bioreactor of 3 m³.

### EXAMPLES

➢ **Characterization of different OW fractions** throughout the composting treatment, following NREL methodology and by gravimetric tests. The substrate chosen is the material generated at an intermediate point of the process (prior to screening, after trench outflow), which provides an adequate amount of carbohydrates and represents a saving by not completing the entire composting process.

**Table 1. Composition of different OW fractions used in development (S1, fraction less than∅ 80 mm; S2 fraction after trench outflow, S3 compost after refining)**

| | **S1.1** | **S1.2** | **S1.3** | **S2.1** | **S2.2** | **S2.3** | **S3.1** | **S3.2** |
|---|---|---|---|---|---|---|---|---|
| **Total solids** | 39.3 | 36.2 | 41.2 | 57.5 | 66.3 | 63.4 | 66.7 | 59.2 |
| **Ashes** | 39.0 | 32.8 | 34.5 | 39.7 | 44.4 | 38.0 | 31.6 | 33.5 |
| **Fats** | 6.4 | 9.5 | 11.1 | 16.3 | 16.2 | 16.5 | 13.0 | 13.9 |
| **Extractive H2O** | 24.0 | 24.9 | 21.3 | 12.0 | 13.0 | 13.7 | 17.2 | 17.6 |
| **Glucan** | 18.7 | 22.6 | 20.1 | 31.0 | 21.0 | 25.0 | 25.2 | 24,.0 |
| **Xylan** | 1.6 | 1.8 | 1.6 | 2.4 | 2.7 | 2.4 | 2.3 | 2.2 |
| **Pectin** | 7.2 | 6.4 | 8.1 | 12.3 | 11.4 | 11.1 | 10.7 | 9.9 |
| **Lignin** | 3.0 | 4.6 | 3.9 | 5.2 | 4.6 | 6.1 | 6.9 | 6.9 |
| **Starch** | 4.7 | 4.9 | 4.6 | 4.9 | 2.4 | 3.0 | 1.9 | 2.1 |
| **Protein** | 9.2 | 14.6 | 8.6 | 5.5 | 6.5 | 4.4 | 5.0 | 6.0 |

➢ **Development of the hydrolysis process** with commercial enzymes: We conducted ydrolysis assays with cellulase combined with amylases, lipases and proteases. Practically in all cases, approximately 20 g/L of glucose and about 5 g/L of xylose are released.
➢ **Hydrolysate tolerance tests.** We tested growth of strains with different concentrations of hydrolysate using the culture media salts proposed to perform the first fermentation assays. We analyzed the supernatant by HPLC. The bacteria used were: *Raoultella planticola* CECT843, and *Bacillus licheniformis* DSM8785. In this case, these bacteria produce 2,3-BD up to a 75% hydrolyzed concentration. The best results were obtained with *R. planticola.*
➢ **Production assays.** Based on the tolerance test results, longer fermentation tests and sampling were carried out to monitor growth and production.

**Table 2. Assay of 2,3-Butanediol production with hydrolysate at 75 or 50% of the OW fraction after trench outflow**

| **Strain** | **Time (h)** | **CFU/mL** | **Glucose (g/L)** | **2,3-Butanediol** |
|---|---|---|---|---|
| ***Raoutella planticola*** | 0 | 18·10⁷ | 15.9 | - |
| | 16 | - | 13.7 | 1.4 |
| | 24 | 27.7·10⁸ | 1.9 | 3.3 |
| **% *hydrolysate: 75*** | 40 | - | - | 5.6 |
| | 48 | 25.7·10⁹ | - | 5.6 |
| | 65 | 49.0·10⁹ | - | 4.6 |
| ***Bacillus licheniformis*** | 0 | 4.7·10⁴ | 10.6 | - |
| | 16 | - | - | 4.3 |
| | 24 | 7.7·10⁸ | - | 2.2 |
| **% *hydrolysate: 50*** | 40 | - | - | 1.0 |
| | 48 | 21.7·10⁸ | - | 0.9 |
| | 65 | 20.7·10⁸ | - | 0.9 |

The best results were obtained with *R. planticola,* so this strain was selected for subsequent trials.

### ➢ Process scale-up.

### - Optimization of the culture medium and operating mode

Tests were performed in flasks by comparing formulated and hydrolyzed media without any supplements. The results are shown in Table 3. The assay was carried out at 30ºC under shaking at 150 rpm. Cell growth was measured by counting viable cells at the beginning and end of the batch (CFU/mL). These results highlight the use of hydrolysate alone, without adding salts, to obtain similar 2,3-BD levels compared to those obtained with formulated media, particularly with the formulated media M1, M2 and M3 defined above. The highest values correspond to M3 medium, reaching 4.8 g/L.

**Table 3. Production of 2,3-Butanediol with R. planticola from MSW hydrolysate after trench outflow**

| **Medium** | **Time (h)** | **CFU/mL** | **Glucose** | **2,3-BD (g/L)** |
|---|---|---|---|---|
| **Hydrolysate** | 0 | 8.3·10⁸ | 21.2 | - |
| | 16 | - | 17.9 | 1.1 |
| | 20 | 2.4·10¹¹ | 15.0 | 2.3 |
| **M1** | 0 | 5.3·10⁸ | 19.5 | - |
| | 16 | - | 16.7 | 1.1 |
| | 20 | 1.0·10¹¹ | 13.6 | 2.8 |
| **M2** | 0 | 6.7·10⁸ | 17.1 | - |
| | 16 | - | 16.0 | 0.5 |
| | 20 | 7.3·10¹⁰ | 13.9 | 1.9 |
| **M3** | 0 | 8.0·10⁸ | 19.4 | - |
| | 16 | - | 14.2 | 2.5 |
| | 20 | 8.0·10⁸ | 9.7 | 4.8 |

The crop feeding strategy using a *fed-batch* system was analyzed. The system was fed by substrate pulses at 16, 24 and 40 hours. The results are shown in Table 4. This strategy allows the amount of substrate available in the medium to be increased without inhibiting the growth of the strain.

The substrate is totally consumed at 48 hours and 2,3-BD production rates are higher than 14 g/L. The same values are obtained from hydrolysate without supplementation of the formulated media, which is highly advantageous in terms of process costs, as it does not require the addition of salts or supplements. In addition, this medium allows for culture growth tracking *in situ* by optical density measurements (OD₆₀₀).

**Table 4. Production of 2,3-Butanediol with R. planticola CECT843 from hydrolysate of OW added by pulses (fed-batch).**

| **Medium** | **Time (h)** | **CFU/mL** | **Glucose (g/L)** | **2,3-BD (g/L)** |
|---|---|---|---|---|
| ***Hydrolysate*** | 0 | 3,3·10⁸ | 22.1 | - |
| | 16 | 1,1·10¹¹ | 14.9 | 3.4 |
| | 24 | 2,4·10⁹ | 14.3 | 5.0 |
| | 40 | 3,0·10¹⁰ | 5.0 | 12.1 |
| | 48 | 3,7·10¹⁰ | 0.6 | 14.7 |
| **M1** | 0 | 7,0·10⁸ | 19.8 | - |
| | 16 | 2,0·10¹⁰ | 13.1 | 3.4 |
| | 24 | 5,0·10¹⁰ | 12.7 | 5.2 |
| | 40 | 3,0·10¹⁰ | 4,.3 | 12.1 |
| | 48 | 1,0·10¹⁰ | 0.6 | 14.5 |
| | 0 | 6,3·10⁸ | 16.6 | - |
| **M2** | 16 | 9,0·10¹⁰ | 10.8 | 3.5 |
| | 24 | 2,5·10⁹ | 9.5 | 6.5 |
| | 40 | 1,0·10¹⁰ | 0.5 | 13.9 |
| | 48 | 1,3·10¹⁰ | 0.5 | 14.4 |
| **M3** | 0 | 4,7·10⁸ | 19.1 | - |
| | 16 | 5,0·10¹⁰ | 12.9 | 3.1 |
| | 24 | 7,7·10¹⁰ | 11.5 | 5.8 |
| | 40 | 3,0·10¹⁰ | 0.49 | 13.1 |
| | 48 | 1,3·10¹⁰ | 0.43 | 14.3 |

### - Production assays of 2,3-BD in sequential saccharification and fermentation (SSF)

Hydrolysate obtained from the S3 substrate (post-refined compost) was used for this assay. It was performed at flask scale with the strain *R. planticola* CECT843. The results showed tolerance of the strain to the hydrolysate without separating the solid fraction, and a satisfactory yield of 2,3-BD production (Table 5).

**Table 5. Production of 2,3-Butanediol with R. planticola CECT843 in SSF with compost hydrolysate.**

| | **Glucose (g/L)** | **2,3-BD (g/L)** | **Rto. (%)** |
|---|---|---|---|
| Sequential saccharification and fermentation flask | 32.5 | 12.5 | 38.5 |

### - Fermentation scale up

### From flask to 1-liter fermenter

As scale-up starting point, a bioreactor with a maximum volume of 1.5 L was used, and 1 L of workload from hydrolysate to 80% (OW after trench outflow) The conditions were as follows:
- Temperature: 30 ⁰ C
- PH: 6.5 at startup and then without control
- Shaking: 325rpm
- Aeration: 1, 5 vvm

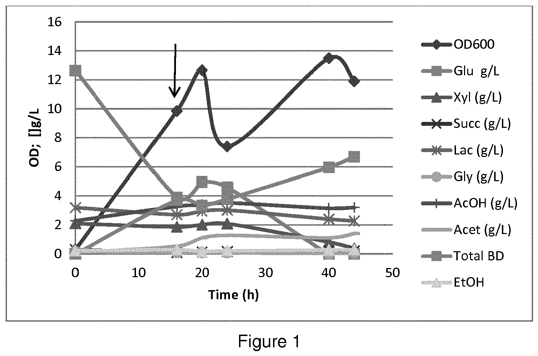

We used *fed-batch* mode for substrate addition by pulses at 16, 21.24 and 41 h. The results of the fermentation are given in Figure 1, which shows a decrease in OD (cell concentration) when pulses were added due to a dilution effect, but which subsequently recovered. Pulse addition to the substrate seems to produce an inhibition effect and stop 2,3BD production, but then it recovers. The main product is the mesoform of 2,3-BD. Hardly any acids are produced, which indicates that the fermentation conditions are adequate. Both glucose and xylose are consumed.

### Addition of concentrated Substrate:

We evaluated the addition of substrate by pulses using a 10 times concentrated hydrolysate, which enabled an increase in glucose concentration in the broth. The fermentation was performed in a bioreactor with a maximum volume of 1.5 L and a 1 L workload with hydrolysate at 80%. The conditions were as follows:
- Temperature: 30⁰C.
- PH: 6.5 at startup and then without control.
- Shaking: 325rpm.
- Aeration 1.5 vvm

We used the strategy of adding concentrated substrate (200 g/L of glucose) in *fed-batch* mode by pulses at 16, 18, 24 and 36 h.

In fermentations adding pulses of concentrated substrate, we can observe that productivity falls after 45 hours despite having a sufficient source of carbon available to achieve the desired yields. By contrast, pH fell to 5.2 during fermentation.

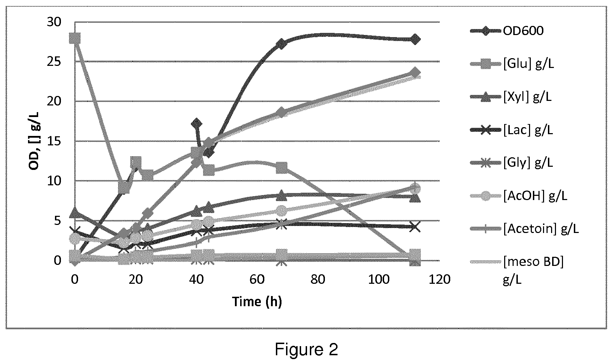

The results of fermentation are shown in Figure 2. In this case a concentration of around 25 g/L was reached.

### Process development and optimization

### - Optimization of environmental conditions

Compared to the results obtained with the different environmental conditions, it is observed that:
- The optimum operating pH is below 7.
- This is in the optimum temperature range for fermentation.
- Moderate oxygen concentrations are required.
- The strain tolerates hydrolysate; however, the optimal amount is around 75-80% for the strain to start growing in the best conditions.

Assessment was also made of the need to control pH during the process, observing that pH must be adjusted to the initial value coinciding with the pulses of substrate.

### - Trials with improved strains of R. planticola.

Subsequently, the improved strains available in the collection at Biopolis were tested to evaluate their tolerance to hydrolysate and production of 2,3-BD, *Raoultella planticola* CECT8158 and *Raoultella planticola* CECT8159. The results show that the improved strains behave in a similar way to the wild strain with the hydrolyzed OW as substrate, and therefore we continued to work with this strain to scale-up fermentation (table 6).

**Table 6. Comparison of the wild strain CECT843 with mutant strains CECT8158 and CECT8159.**

| **Strain** | **Time (h)** | **CFU/mL** | **Glucose (g/L)** | **2,3 -BD** |
|---|---|---|---|---|
| *R. planticola* CECT 8158 | 0 | 5.2·10⁸ | 22.4 | - |
| | 16 | 3.1·10¹⁰ | 15.2 | 4.1 |
| | 24 | 4.3·10¹⁰ | 14.8 | 5.8 |
| | 40 | 3.6·10¹⁰ | 6.1 | 12.6 |
| | 48 | 7.4·10¹⁰ | 0.4 | 14.1 |
| *R. planticola* CECT | 0 | 6,3·10⁸ | 22.6 | - |
| | 16 | 3.7·10¹⁰ | 14.7 | 3.9 |
| | 24 | 5.1·10¹⁰ | 14.3 | 5.3 |
| | 40 | 4.3·10¹⁰ | 5.8 | 11.9 |
| | 48 | 6.9·10¹⁰ | 0.5 | 14.4 |
| *R. planticola* CECT843 | 0 | 4.7·10⁸ | 19.1 | - |
| | 16 | 5.0·10¹⁰ | 12.9 | 3.1 |
| | 24 | 7.7·10¹⁰ | 11.5 | 5.8 |
| | 40 | 3.0·10¹⁰ | 0.49 | 13.1 |
| | 48 | 1.3·10¹⁰ | 0.43 | 14.3 |

### - Fermentation optimization in the bioreactor-pH adjustment.

Finally, we decided to follow a strategy of adding the concentrated substrate (200 g/L of glucose) and of NaOH pulses to maintain the pH levels during fermentation. A bioreactor with a maximum volume of 1.5 L was used, and 1 L of workload with 80% hydrolysate. The conditions were as follows:
- Temperature: 30⁰C.
- Initial pH: 6.5
- Shaking: 325 rpm.
- Aeration: 1.5 vvm

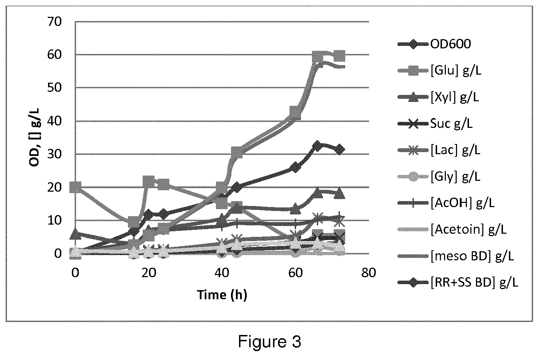

We used the strategy of adding concentrated substrate (200 g/L of glucose) and NaOH in *fed-batch* mode by pulses at 16, 24, 41 and 48 h. During fermentation pH was maintained at around 6. The results obtained following this fermentation strategy (Figure 3) show values of 50 g/L of 2.3-BD at around 70 hours of fermentation. Thus, fermentation scale was increased (20 L).

### - Pilot scale-up: from a 1.5 L bioreactor to a 20 L fermenter.

In this case, the substrate concentration required is at 10 times larger volumes. The fermentation conditions using 80% hydrolysate were as follows:
- Temperature: 30⁰C.
- pH 6.5 at start up.
- Shaking: 200 rpm.
- Aeration: 0.5 vvm

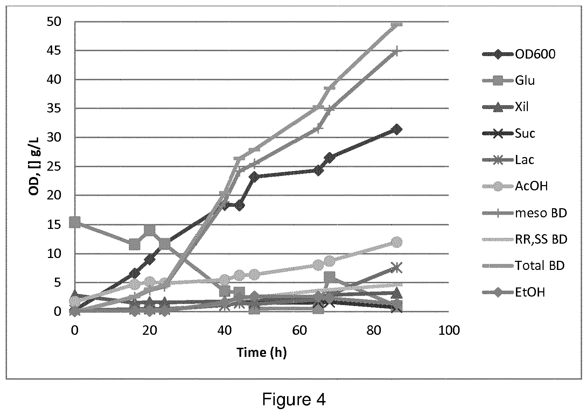

The strategy employed was to add concentrated substrate (200 g/L of glucose) and NaOH in *fed-batch* mode by pulses at 16, 24, 40 and 65 h. During fermentation pH was maintained at around 6. The results obtained during fermentation are shown in Figure 4. Good results were obtained in this scale-up step, since the target concentration of 50 g/L of 2,3-BD was achieved and the results obtained in the optimization step were reproduced.

### - Demonstration of scale-up at a 3 m³ scale

In this case, a hydrolysate was prepared at a 3 m³ scale with S3 substrate (OW after refinement) and fractions were formulated and concentrated for pulse feeding.

The fermentation conditions using 80% hydrolysate were as follows:
- Temperature: 30⁰C.
- pH 6.5 at start up.
- Shaking: 200 rpm.
- Aeration: 0.5 vvm

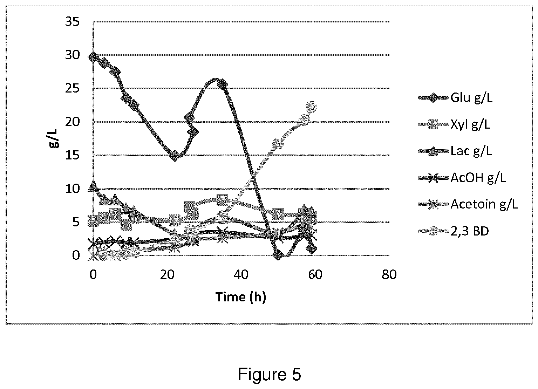

The inoculum used was prepared at a 100 L scale, in which the culture medium was the same hydrolysate in order to adapt the strain to the substrate. The strategy employed was to add concentrated substrate (200 g/L of glucose) and NaOH in *fed-batch* mode by pulses at 12, 24, 29, 36, 48 and 54 h. During fermentation pH was maintained at around 6. The results obtained during fermentation are shown in Figure 5.

## Claims

1. Procedure to obtain 2,3-Butanediol from a hydrolyzed substrate of OW (organic waste) that comprises the fermentation of this substrate with a strain of *Raoultella planticola,* wherein:
OW is MSW (municipal solid waste), BMSW (biodegradable fraction of municipal solid wastes, stabilized or unstabilized), sewage sludge from WWPP (waste water purification plants), agricultural and livestock waste (such as slurry), waste from the agro-alimentary industry, and mixtures thereof, **characterized in that** OW is generated in urban areas,
the strain of *Raoultella planticola* is CECT843, CECT8158 or CECT8159,
the hydrolysate of OW is used as a substrate at a concentration of between 50% and 100%, and
the OW substrate is formulated with the culture media M1, M2 or M3, wherein:
M1 consisting of 4.4 g/L K₂HPO₄, 1.3 g/L KH₂PO₄, 2 g/L (NH₄)₂SO₄, 0.2 g/L MgSO₄, 1.0 g/L yeast extract, 1 mL/L trace elements solution [70 mg/L ZnCl₂, 0.1 g/L MnCl₂·4H₂O, 60 mg/L H₃BO₃, 0.2 g CoCl₂H₂O, 20 mg CuCl₂·2H₂O, 25 mg NiCl₂·6H₂O, 35 mg Na₂MoO₄·2H₂O, 0.9 mL/L HCl];
M2 consisting of 5.0 g/L yeast extract, 5.0 g/L triptone, 7.0 g/L KH₂PO₄, 7.0 g/L K₂HPO₄, 1.0 g/L (NH₄)₂SO₄, 0.25 g/L MgSO₄·7H₂O, 0.12 g/L NaMoO₄·7H₂O, 0.021 g/L CaCl₂·2H₂O, 0.029 g/L CoCl₂·6H₂O, 0.039 g/L Fe(NH₂)₂SO₄·6H₂O, 2 mg/L nicotininic acid, 0.172 mg/L Na₂SeO₃, 0.02 mg/L NiCl₂, 10 mL/L trace elements solution [0.5 g/L Na₂EDTA, 0.5 g/L MnCl₂·4H₂O, 0.1 g/L H₃BO₃, 1.0 mg/L CuCl₂·2H₂O]; and
M3 consisting of 0.75 g/L KCI, 1.38 g/L NaH₂PO₄·2H₂O, 5.35 g/L (NH₄)₂SO₄, 0.28 g/L Na₂SO₄, 0.26 g/L MgSO₄·7H₂O, 0.42 g/L citric acid, 2 g/L yeast extract, 0.3 mL/L trace elements solution [34.2 g/L ZnCl₂, 2.7 g/L FeCl₃·6H₂O, 10 g/L MnCl₂·4H₂O, 0.85 g/L CuCl₂·2H₂O, 0.3 g/L H₃BO₃, 23.8 g/L CoCl₂·6H₂O].

2. The procedure according to claim 1, where the temperature at which it is performed is between 25°C and 35°C.

3. The procedure according to any of the claims 1 or 2, **characterized by** being carried out under shaking at between 100 rpm and 400 rpm.

4. The procedure according to any one of the claims 1 to 3, **characterized by** being carried out at a pH of less than 7.

5. The procedure according to any one of the claims 1 to 4, **characterized by** being carried out with aeration of between 0.7 vvm and 2.0 vvm.

6. The procedure according to any one of the claims 1 to 5, which comprises a culture feeding step by means of fed-batch mode.

7. The procedure according to any of the claims 1 to 6, where the substrate feeding by fed-batch is carried out with pulses of substrate at different time intervals.

## Patentansprüche

1. Verfahren zur Gewinnung von 2,3-Butandiol aus einem hydrolysierten Substrat von OA (organic waste, organischen Abfällen), das die Fermentation dieses Substrats mit einem Stamm von OA *Raoultella planticola umfasst,* wobei:
OA Siedlungsabfälle (municipal solid waste, MSW), BMSW (biodegradable fraction of municipal solid wastes, biologisch abbaubare Fraktion von Siedlungsabfällen, stabilisiert oder nicht stabilisiert), Klärschlamm aus WWPP (waste water purification plants, Abwasserreinigungsanlagen),
landwirtschaftliche und tierische Abfälle (z. B. Gülle), Abfälle aus der Agrar- und Ernährungswirtschaft Industrie und Mischungen davon sind, **dadurch gekennzeichnet, dass** OA in städtischen Gebieten erzeugt wird,
der Stamm von *Raoultella planticola* CECT843, CECT8158 oder CECT8159 ist, das Hydrolysat von OA als Substrat in einer Konzentration zwischen 50 % und 100 % verwendet wird, und
das OA-Substrat mit den Kulturmedien M1, M2 oder M3 formuliert wird, wobei:
M1 aus 4,4 g/l K₂HPO₄, 1,3 g/l KH₂PO₄, 2 g/l (NH₄)₂SO₄, 0,2 g/l MgSO₄, 1,0 g/l Hefeextrakt, 1 ml/l Spurenelementlösung [70 mg/l ZnCl₂, 0,1 g/l MnCl₂·4H₂O, 60 mg/l H₃BO₃, 0,2 g CoCl₂H₂O, 20 mg CuCl₂·2H₂O, 25 mg NiCl₂·6H₂O, 35 mg Na₂MoO₄·2H₂O, 0,9 ml/l HCl] besteht;
M2 aus 5,0 g/l Hefeextrakt, 5,0 g/l Tripton, 7,0 g/l KH₂PO₄, 7,0 g/lK₂HPO₄, 1,0 g/l (NH₄)₂SO₄, 0,25 g/l MgSO₄·7H₂O, 0,12 g/l NaMoO₄·7H₂O, 0,021 g/l CaCl₂·2H₂O, 0,029 g/l CoCl₂·6H₂O, 0,039 g/l Fe(NH₂)₂SO₄·6H₂O, 2 mg/l Nicotinsäure, 0,172 mg/l Na₂SeO₃, 0,02 mg/l NiCl₂, 10 ml/L Spurenelementlösung [0,5 g/l Na₂EDTA, 0,5 g/l MnCl₂·4H₂O, 0,1 g/l H₃BO₃, 1,0 mg/l CUCl₂·2H₂O] besteht; und
M3 aus 0,75 g/l KCI, 1,38 g/l NaH₂PP₄·2H₂O, 5,35 g/l (NH₄)₂SO₄, 0,28 g/l Na₂SO₄, 0,26 g/l MgSO₄·7H₂O, 0,42 g/l Citronensäure, 2 g/l Hefeextrakt, 0,3 ml/l Spurenelementlösung [34,2 g/l ZnCl₂, 2,7 g/l FeCl₃·6H₂O, 10 g/l MnCl₂·4H₂O, 0,85 g/l CuCl₂·2H₂O, 0,3 g/l H₃BO₃, 23,8 g/l CoCl₂·6H₂O] besteht.

2. Verfahren nach Anspruch 1, wobei die Temperatur, bei der es durchgeführt wird, zwischen 25 °C und 35 °C liegt.

3. Verfahren nach einem der Ansprüche 1 oder 2, **dadurch gekennzeichnet, dass** es unter Schütteln bei zwischen 100 U/min und 400 U/min durchgeführt wird.

4. Verfahren nach einem der Ansprüche 1 bis 3, **dadurch gekennzeichnet, dass** es bei einem pH-Wert von weniger als 7 durchgeführt wird.

5. Verfahren nach einem der Ansprüche 1 bis 4, **dadurch gekennzeichnet, dass** es mit einer Belüftung zwischen 0,7 vvm und 2,0 vvm durchgeführt wird.

6. Verfahren nach einem der Ansprüche 1 bis 5, das einen Kulturfütterungsschritt mittels Fed-Batch-Modus umfasst.

7. Verfahren nach einem der Ansprüche 1 bis 6, wobei die Substratzufuhr per Fed-Batch mit Substratimpulsen in unterschiedlichen Zeitintervallen durchgeführt wird.

## Revendications

1. Procédure pour obtenir du 2,3-butanediol à partir d'un substrat hydrolysé de DO (déchet organique) qui comprend la fermentation de ce substrat avec une souche de *Raoultella planticola,* dans laquelle :
DO est des DSM (déchets ménagers), une BDSM (fraction biodégradable de déchets solides municipaux, stabilisée ou non stabilisée), des boues d'égouts de STEP (stations d'épuration des eaux usées), des déchets agricoles et d'élevage (tels que le lisier), des déchets de l'industrie agro-alimentaire, et des mélanges de ceux-ci, **caractérisée en ce que** le DO est généré dans des zones urbaines,
la souche de *Raoultella planticola* est CECT843, CECT8158 ou CECT8159, l'hydrolysat de DO est utilisé en tant que substrat à une concentration d'entre 50 % et 100 %, et
le substrat de DO est formulé avec les milieux de culture M1, M2 ou M3, dans laquelle :
M1 consistant en 4,4 g/l de K₂HPO₄, 1,3 g/l de KH₂PO₄, 2 g/l de (NH₄)₂SO₄, 0,2 g/l de MgSO₄, 1,0 g/l d'extrait de levure, 1 ml/l de solution d'oligo-éléments [70 mg/l de ZnCl₂, 0,1 g/l de MnCl₂ 4H₂O, 60 mg/l de H₃BO₃, 0,2 g de CoCl₂H₂O, 20 mg de CuCl₂ 2H₂O, 25 mg de NiCl₂ 6H₂O, 35 mg de Na₂MoO₄ 2H₂O, 0,9 ml/l de HCl] ;
M2 consistant en 5,0 g/l d'extrait de levure, 5,0 g/l de triptone, 7,0 g/l de KH₂PO₄, 7,0 g/l de K₂HPO₄, 1,0 g/l de (NH₄)₂SO₄, 0,25 g/l de MgSO₄ 7H₂O, 0,12 g/l de NaMoO₄ 7H₂O, 0,021 g/l de CaCl₂ 2H₂O, 0,029 g/l de CoCl₂ 6H₂O, 0,039 g/l de Fe(NH₂)₂SO₄ 6H₂O, 2 mg/l d'acide nicotinique, 0,172 mg/l de Na₂SeO₃, 0,02 mg/l de NiCl₂, 10 ml/l de solution d'oligo-éléments [0,5 g/l de Na₂EDTA, 0,5 g/l de MnCl₂ 4H₂O, 0,1 g/l de H₃BO₃, 1,0 mg/l de CUCl₂ 2H₂O] ; et
M3 consistant en 0,75 g/l de KCI, 1,38 g/l de NaH₂PP₄ 2H₂O, 5,35 g/l de (NH₄)₂SO₄, 0,28 g/l de Na₂SO₄, 0,26 g/l de MgSO₄ 7H₂O, 0,42 g/l d'acide citrique, 2 g/l d'extrait de levure, 0,3 ml/l de solution d'oligo-éléments [34,2 g/l de ZnCl₂, 2,7 g/l de FeCl₃ 6H₂O, 10 g/l de MnCl₂ 4H₂O, 0,85 g/l de CuCl₂ 2H₂O, 0,3 g/l de H₃BO₃, 23,8 g/l de CoCl₂ 6H₂O].

2. Procédure selon la revendication 1, où la température à laquelle elle est effectuée est entre 25 °C et 35 °C.

3. Procédure selon l'une quelconque des revendications 1 ou 2, **caractérisée en ce qu'**elle est réalisée sous agitation entre 100 tr/min et 400 tr/min.

4. Procédure selon l'une quelconque des revendications 1 à 3, **caractérisée en ce qu'**elle est réalisée à un pH inférieur à 7.

5. Procédure selon l'une quelconque des revendications 1 à 4, **caractérisée en ce qu'**elle est réalisée avec une aération d'entre 0,7 vvm et 2,0 vvm.

6. Procédure selon l'une quelconque des revendications 1 à 5, qui comprend une étape d'alimentation de culture au moyen d'un mode à alimentation programmée.

7. Procédure selon l'une quelconque des revendications 1 à 6, où l'alimentation de substrat par alimentation programmée est réalisée avec des impulsions de substrat à différents intervalles de temps.
